# EUROPEAN PATENT SPECIFICATION

(11) **EP 1 324 669 B1**
(45) Date of publication and mention of the grant of the patent: **29.03.2006**
(21) Application number: 01974563.7
(22) Date of filing: 03.10.2001
(51) Int. Cl.: A23J 1/16, A23K 1/16

(54) **MODIFIED CORN STEEP LIQUOR PRODUCT**
MODIFIZIERTES MAISQUELLWASSERPRODUKT
PRODUIT MODIFIE A BASE D'EXTRAIT SOLUBLE DE MAIS

(30) Priority: 03.10.2000 ZA 200005371
(43) Date of publication of application: 09.07.2003
(73) Proprietor: SA Bioproducts (PTY) Ltd., 4126 Umbogintwini (ZA)
(72) Inventor: VAN WALSEM, Hendrik, Johan, Cambridge, MA 02142-1126 (US); SASKA, Michael, Baton Rouge, CA 70808 (US)
(74) Representative: Curtis, Philip Anthony
(86) International application number: PCT/IB2001/001822
(87) International publication number: WO 2002/028196

(56) References cited:
- EP-A- 0 041 894
- GB-A- 740 205
- PATENT ABSTRACTS OF JAPAN vol. 1998, no. 08, 30 June 1998 (1998-06-30) & JP 10 075795 A (OJI KOONSUTAAC KK), 24 March 1998 (1998-03-24)
- DATABASE WPI Section Ch, Week 200048 Derwent Publications Ltd., London, GB; Class D13, AN 2000-526587 XP002193269 & JP 11 266846 A (MORINAGA & CO LTD), 5 October 1999 (1999-10-05)

## Description

### BACKGROUND OF THE INVENTION

THIS invention relates to a modified corn steep liquor product.

Myo-inositol (also known as inositol, muscle sugar, and hexahydroxycyclohexane) is an important growth factor considered by some as part of the B vitamin group (Szmant, "Industrial utilization of renewable resources'', Technomic Publication Company, Lancaster, PA, 1986). It is found in plants both in the free form (in soya and sugar cane for instance) and also as a hexaphosphate, phytin, (in corn for instance) and also as a phosphatidyl derivative, which is a component of vegetable lecithins.

The main applications of myo-inositol are in aquaculture, pet food, animal feed and in the developing markets in health and nutrition.

Com steep liquor is the traditional industrial source of myo-inositol (e.g Artz and Hach, "Process for refining crude inositol containing solutions and for recovering inositol therefrom", US Patent No 2,615,053, 21 October 1952). In this process, phytin is first precipitated as a calcium salt and then hydrolysed with a strong acid. The production of myo-inositol in this fashion was stopped in the USA in about 1987 because of high production costs. Thus, supplies of myo-inositol now come from China (where it is made from corn steep liquor) and also from Japan (where it is made from rice bran).

US Patent No 3,270,064 to Toyo Koatsu Industries Inc, describes an improvement to the chemical precipitation and hydrolysis process by using a soluble base to raise the pH (e.g ammonia or caustic) and precipitate the phytin. This is followed by hydrolysis under heat and pressure in the presence of an alkaline earth metal carbonate such as magnesium carbonate. Benefits claimed are elimination of complexing reactions and improved purity of the final product. Chemical consumption is still high and the use of caustic or ammonia detracts from the value of the remaining material, as it is not possible to use it as feed.

Because of the chemicals involved in the precipitation and hydrolysis of phytin, any source of the free form of myo-inositol will be at an advantage, provided an efficient way can be found to separate the myo-inositol from the other components. To this effect two processes have been patented, viz. US Patent No 5,096,594 to Rabinowitz and US Patent No 4,482,761 to Chao and Sherman. One process is based on the use of cation exchange resins in Ca²⁺ or Pb²⁺ form with aqueous dimethyl sulfoxide as the mobile phase and the other is based on zeolite molecular sieves. The former process has the disadvantages of using an organic solvent and the use of lead in a food product is entirely unacceptable. The latter process is not very efficient.

JP-A-10075795 discloses a composition comprising inositol phosphoric acids.

Myo-inositol cannot be efficiently separated from monosaccharides (glucose and particularly fructose) on cation exchange resins in monovalent (Na⁺ or K⁺) or divalent (Ca²⁺) form. The use of strong base anion resins to separate myoinositol from sugar steams has been demonstrated as a feasible option for molasses type streams from the sugar industry (Saska, "Industrial production of inositol: A by-product from the cane molasses desugarization process" 1997, Int. Sugar Jnl., (99): 480-484). -

Com steep liquor cannot be used directly as a feed for sensitive feed applications such as small animals and especially weaning piglets, as the high level of potassium and other salts promote scours and other digestive disorders. Nevertheless, corn steep liquor is a high quality protein source, rich in highly available soluble protein and is of interest in specialist feed applications, provided the problematic components can be separated therefrom.

### SUMMARY OF THE INVENTION

According to the invention there is provided a modified corn steep liquor product which is characterised in that:
it is substantially ash free, i.e free of inorganic salts, especially potassium salts;
it contains from 7,5% by weight on a dry basis of myo-inositol; and
it contains from 60% by weight on a dry basis of proteins.

The modified corn steep liquor product may be prepared from a corn steep liquor which includes suspended solids, high molecular weight compounds, phytic acid, and metal values (Mg, Ca, Fe, Mn, Zn, Na and K), generally in the form of metal ions, by a method which includes the steps of:
(a) subjecting the corn steep liquor to ultrafiltration to remove the suspended solids and high molecular weight compounds;
(b) treating the product of step (a) with a phytase enzyme to convert the phytic acid to inositol-mono-phosphate;
(c) treating the product of step (b) with an acid phosphatase enzyme to convert the inositot-monorphosphate to myo-inositol;
(d) adjusting the pH of the product of step (c) to from 7 to 9 inclusive using a soluble base which causes most of all or the Mg values to precipitate, and removing the precipitate; and
(e) contacting the product of step (d) with a strong acid cation chromatographic resin to produce a first fraction containing most of the remaining metal values, and a second fraction containing most of the myo-inositol, the second fraction being the modified corn steep liquor product.

### BRIEF DESCRIPTION OF THE DRAWINGS

- **Figure 1**: is a chromatogram from the Experimental Work; and
- **Figure 2**: is a flow diagram illustrating a continuous simulated moving bed from the Experimental Work.

### DESCRIPTION OF EMBODIMENTS

The crux of the invention is a modified corn steep liquor product which is characterised in that it is substantially ash free, i.e free of inorganic salts, especially potassium salts; it contains from 7.5% by weight on a dry basis of myo-inositol; and it contains from 60% by weight on a dry basis of proteins.

The product preferably contains from 10% by weight on a dry basis of myo-inositol and from 75% by weight on a dry basis of proteins.

The product also preferably contains from 5% by weight on a dry basis of sugars.

The modified corn steep liquor product of the invention is of application as an animal feed.

The modified corn steep liquor product may be prepared by a method which is described in more detail with reference to the following experimental work:

### Experimental Work

### Step (a) - Ultrafiltration

Crude corn steep liquor (CSL) from a corn wet mill was diluted to 30% (m/m) solids and fed to a Niro ultrafiltration plant with the following operating conditions:
Advanced Mambrane Technology Inc. (AMT) spiral wound PVDF membranes of 30 000 kD,
Cross flow velocity 2-4 m/s using 80 mils spacers (2 mm). Pressure drop 1 Bar,
Trans-membrane pressure (TMP) of 4 - 5 Bar,
Concentration ratio (retentate/feed) of 0,5.
Turbidity of permeate (suspended solids) less than 10 NTU (nephelometric turbidity units) as measured by nephelometer.
Feed pH of 4,0,
Temperature of 40°C.
The result is a product from which suspended solids and high molecular weight compounds, which would lead to fouling of the resin in step (e), are removed.

### Step (b)- Phytase Treatment

The myo-inositol in the clarified CSL from step (a) is essentially present as phytic acid or as a salt thereof. Being a phosphate containing product, this product would follow the ash fraction in any separation, and thus the phytic acid must be converted to free myo-inositol before separation of the ash fraction. This is achieved by enzymatic hydrolysis of the phytic acid to inositol-mono-phosphate using a phytase enzyme. The treatment conditions are as follows:
Enzyme used: BASF Natuphos as described in BASF product brochure in free form,
Enzyme dosage: 500 FTU (phytase activity units) per kg CSL (as 30% solids),
Optimum pH: 3,0 to 4,0 to ensure adequate phytase activity and also shelf life of the CSL,
Temperature of 40°C,
Residence time: 12 hours.

The resulting product contains inositol-mono-phosphate. An analysis of a typical product from step (b) is as follows:

| Component | Units | Value |
|---|---|---|
| **General analyses** | | |
| Density | Kg/m³ | 1150 |
| PH | | 3,0 |
| Dry matter (Brix) | % (m/m) | 31,0 |

| **Organic components** | | |
|---|---|---|
| Glucose | % m/m | 4,21 |
| Frutose | % m/m | 1,68 |
| D-lactate | % m/m | 5,05 |
| L-lactate | % m/m | 5,61 |
| Phytic Acid | % m/m | <1 |
| Inositol-mono-phosphate | % m/m | 5,61 |

| Amino Acids | | |
|---|---|---|
| Threonine | % m/m | 0,45 |
| Methionine | % m/m | 0,45 |
| Valine | % m/m | 1,57 |
| Alanine | % m/m | 2,69 |
| Glycine | % m/m | 0,34 |
| Aspartate | % m/m | 0,17 |
| Glutamate | % m/m | 0,59 |
| Serine | % m/m | 0,50 |
| Tyrosine | % m/m | 0,28 |
| Arginine | % m/m | 0,76 |
| Iso-leucine | % m/m | 0,45 |
| Leucine | % m/m | 1,43 |
| Histidine | % m/m | 0,14 |
| Cysteine | % m/m | not measured |
| Phenylalanine | % m/m | 0,84 |

| **Protein composition (dry basis)** | | |
|---|---|---|
| Total free amino acids | % (m/m) | 10,0 |
| TKN protein | % (m/m) | 42,0 |

| **Minerals** | | |
|---|---|---|
| Mg | ppm (m/m) | 14516 |
| Ca - | ppm (m/m) | 645 |
| Fe | ppm (m/m) | 210 |
| Mn | ppm (m/m) | 113 |
| Zn | ppm (m/m) | 226 |
| Na | ppm (m/m) | 684 |
| K | % m/m | 6,45 |
| Chlorides | % m/m | 0,98 |
| Free phosphates | % m/m | 1,21 |
| Sulphates | % m/m | 7,43 |

All analyses are expressed on a wet basis unless otherwise stated

### Step (c) - Acid Phosphatase Treatment

An acid phosphatase enzyme is used to hydrolyse the inositol-mono-phosphate containing a single phosphate group to free myo-inositol and the free phosphate. The acid phosphatase treatment conditions are as follows:
Enzyme used: Sumizyme PM-L from the Shin Nihon Company (activity 3000u/g),
Dosage: 1 unit of activity per g of CSL (as 30% solids),
Temperature of 65°C and pH of 4,0 to 4,5,
Residence time: 24 hours,
Conversion achieved: >98% as determined by inositol and IMP measurement by HPL.C.

### Step (d) - pH Adjustment and Removal of Mg Values

It is desirable to conduct the chromatographic resin separation step (Step (e)) without any divalent cations being present. It is well known that these cations have a negative impact on the resolution of the chromatographic separation.

Thus, to avoid this problem, the product of step (c) has its pH adjusted to a value between 7,5 and 8,5 using ammonia, to cause precipitation of struvite.

The precipitate was collected and subjected to an XRF analysis which confirmed the material as being struvite. Analysis of N, Mg and PO₄ using wet chemical assays confirmed the presence of these compounds in the exact stoichiometric quantities to be expected for pure struvite. It was also confirmed that there was very little K (±0,3%) and no Na, Cl, SO₄ or heavy metals present and that the purity was in excess of 99%.

Struvite finds application as a slow release fertiliser.

The clear filtrate was also analysed and it was confirmed that the Mg values were essentially completely removed. This product was then passed on to the next step.

### Step (e) - Chromatographic Separation

The separation was conducted in a single column with the following operating conditions:
Column: 100 cm length with 1 cm diameter,
Resin: Rohm and Haas CR1320 chromatographic resin,
Temperature: 60°C,
Resin volume: 78,5 ml,
Injection volume: 0,01 BV,
Flow rate: 3 ml/min of water (2,25 BV/h or roughly 2,3 m/h velocity),
Detector: On line refractive index (Waters 400).

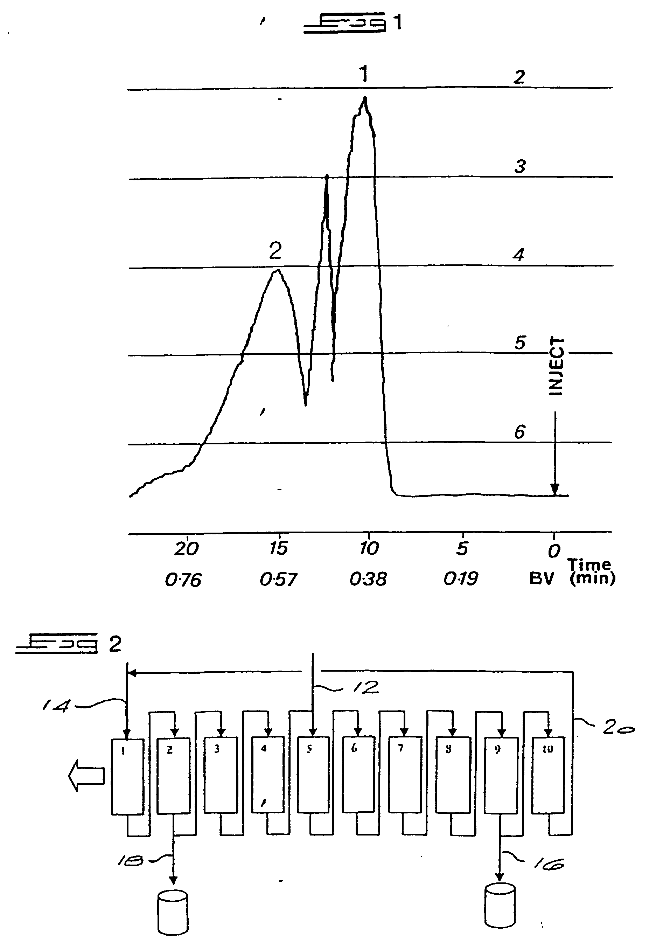

Figure 1 is a chromatogram which shows the separation achieved as follows:

| | |
|---|---|
| Void volume: | 0,32 BV |
| Fraction 1 (raffinate or ash) peak | 0,40 BV |
| Fraction 2 (myo-inositol rich) peak | 0,58 BN |

The peak marked 1 is the ash fraction peak, and the peak marked 2 is the myo-inositol rich fraction peak.

HPLC analyses indicated that the ash fraction contained the lactate, sulphate, phosphate and chloride values and all the cations.

Dry matter determinations indicated that the total solids of the CSL was split equally between fractions 1 and 2, with all the myo-inositol reporting to fraction 2. The concentration of myo-inositol was 10% on a dry matter basis.

In order to optimise the chromatographic separation it is desirable to operate a continuous device such as a simulated moving bed (SMB). In this way it is possible to optimise the eluent and feed flows to achieve the best separation with minimum dilution, whilst maintaining steady operation by effectively maintaining the separation peaks in a stationary position. This means that the two fractions can be extracted from fixed ports as is illustrated in Figure 2 which is described in more detail below.
The design flowrates as indicated were determined from the batch chromatogram (pulse test) discussed above, using standard design and scale-up calculations as used by Advanced Separation Technologies (a division of Calgon Carbon) to design separations based on their Isep rotating carousel system. The design criteria are as follows:

| | |
|---|---|
| Resin volume: | 2,2 litres in total (220 ml per column) |
| Step time: | 4min |
| Resin flow: | 55 ml/min |
| Linear velocity: | 7,5 m/h (safe value for resin of 320 micron) |
| Bed depth: | 0,45 m |
| Feed rate: | 0,1 BV/h |
| Slow Product: | Fraction 2 of Figure 2 being the myo-inositol enriched fraction |
| Fast Product: | Fraction 1 of Figure 2 being the ash containing fraction. |

Referring to Figure 2, there is shown a pilot set-up for continuous separation of myo-inositol enriched CSL. -

The product of step (d) is fed into port 5 of the apparatus via a line 12, at a feed rate of 4 ml/min. Water is fed into port 1 of the apparatus via a line 12, at a feed rate of 27,5 ml/min.

Fraction 1 is removed from the apparatus at port 9 via a line 16, at a rate of 9,35 ml/min.

Fraction 2 is removed from the apparatus at port 2 via a line 18 at a rate of 22,15 ml/min.

Product from port 10 is recycled via a line 20 to the water inlet line 14. The numbers 1 to 10 in the drawing refer to stationary ports. Each of the ten columns containing the resin in the apparatus moves from port to port with a step time of 4 minutes. At each step, each column moves to the next port, and in this way continuous countercurrent resin flow is achieved. The resin flow is 220 ml resin/4 min, or 55 ml/min.

## Claims

1. A modified corn steep liquor product is **characterised in that**:
(1) it is substantially free of inorganic salts;
(2) it contains from 7.5% by weight on a dry basis of myo-inositol; and
(3) it contains from 60% by weight on a dry basis of proteins.

2. A product according to claim 1 which is **characterised in that**: (2) it contains from 10% by weight on a dry basis of myo-inositol.

3. A product according to claim 1 or claim 2 which is **characterised in that**: (3) it contains from 75% by weight on a dry basis of proteins.

4. A product according to any one of claims 1 to 3 **characterised in that**: (4) it contains from 5% by weight on a dry basis of sugars.

5. A product according to any one of claims 1 to 4 for use as an animal feed.

6. An animal feed comprising or containing a modified corn steep liquor product which is **characterised in that**:
(1) it is substantially free of inorganic salts;
(2) it contains from 7.5%-by weight on a dry basis of myo-inositol; and
(3) it contains from 60% by weight on a dry basis of proteins.

7. An animal feed according to claim 6 wherein the product is
**characterised in that**:
(2) it contains from 10% by weight on a dry basis of myo-inositol.

8. An animal feed according to claim 6 or claim 7 wherein the product is
**characterised in that**:
(3) it contains from 75% by weight on a dry basis of proteins.

9. An animal feed according to any one of claims 6 to 8 wherein the product is **characterised in that**:
(4) it contains from 5% by weight on a dry basis of sugars.

## Revendications

1. Produit modifié à base d'extrait soluble de maïs
**caractérisé en ce que** :
(1) il est pratiquement complètement exempt de sels inorganiques ;
(2) il contient à partir de 7,5 % en poids sur une base sèche de myo-inositol ; et
(3) il contient à partir de 60 % en poids sur une base sèche de protéines.

2. Produit selon la revendication 1 qui est
**caractérisé en ce que** :
(2) il contient à partir de 10 % en poids sur une base sèche de myo-inositol.

3. Produit selon la revendication 1 ou la revendication 2 qui est **caractérisé en ce que** :
(3) il contient à partir de 75 % en poids sur une base sèche de protéines.

4. Produit selon l'une quelconque des revendications 1 à 3 **caractérisé en ce que** :
(4) il contient à partir de 5 % en poids sur une base sèche de sucres.

5. Produit selon l'une quelconque des revendications 1 à 4 destiné à l'utilisation comme aliment pour animaux.

6. Aliment pour animaux comprenant ou contenant un produit modifié à base d'extrait soluble de maïs qui est
**caractérisé en ce que** :
(1) il est pratiquement complètement exempt de sels inorganiques ;
(2) il contient à partir de 7,5 % en poids sur une base sèche de myo-inositol ; et
(3) il contient à partir de 60 % en poids sur une base sèche de protéines.

7. Aliment pour animaux selon la revendication 6 dans lequel le produit est **caractérisé en ce que** :
(2) il contient au moins 10 % en poids sur une base sèche de myo-inositol.

8. Aliment pour animaux selon la revendication 6 ou la revendication 7 dans lequel le produit est **caractérisé**
**en ce que** :
(3) il contient à partir de 75 % en poids sur une base sèche de protéines.

9. Aliment pour animaux selon l'une quelconque des revendications 6 à 8 dans lequel le produit est **caractérisé**
**en ce que** :
(4) il contient à partir de 5 % en poids sur une base sèche de sucres.

## Patentansprüche

1. Modifiziertes Maisquellwasserprodukt, das **dadurch gekennzeichnet ist, dass** es
(1) im Wesentlichen frei von anorganischen Salzen ist,
(2) in der Trockenmasse mindestens 7,5 Gew.% myo-Inosit enthält,
(3) in der Trockenmasse mindestens 60 Gew.% Proteine enthält.

2. Produkt nach Anspruch 1, das **dadurch gekennzeichnet ist, dass** es
(2) in der Trockenmasse mindestens 10 Gew.% myo-Inosit enthält.

3. Produkt nach Anspruch 1 oder 2, das **dadurch gekennzeichnet ist, dass** es
(3) in der Trockenmasse mindestens 75 Gew.% Proteine enthält.

4. Produkt nach einem der Ansprüche 1 bis 3, **dadurch gekennzeichnet, dass** es
(4) in der Trockenmasse mindestens 5 Gew.% Zucker enthält.

5. Produkt nach einem der Ansprüche 1 bis 4 zur Benutzung als ein Tierfutter.

6. Tierfutter, aufweisend oder enthaltend ein modifiziertes Maisquellwasserprodukt, das **dadurch gekennzeichnet ist, dass** es
(1) im Wesentlichen frei von anorganischen Salzen ist,
(2) in der Trockenmasse mindestens 7,5 Gew.% myo-Inosit enthält und
(3) in der Trockenmasse mindestens 60 Gew.% Proteine enthält.

7. Tierfutter nach Anspruch 6, wobei das Produkt **dadurch gekennzeichnet ist, dass** es
(2) in der Trockenmasse mindestens 10 Gew.% myo-Inosit enthält.

8. Tierfutter nach Anspruch 6 oder 7, wobei das Produkt **dadurch gekennzeichnet ist, dass** es
(3) in der Trockenmasse mindestens 75 Gew.% Proteine enthält.

9. Tierfutter nach einem der Ansprüche 6 bis 8, wobei das Produkt **dadurch gekennzeichnet ist, dass** es
(4) in der Trockenmasse mindestens 5 Gew.% Zucker enthält.
